# EUROPEAN PATENT SPECIFICATION

(11) **EP 2 582 690 B1**
(45) Date of publication and mention of the grant of the patent: **25.07.2018**
(21) Application number: 11795304.2
(22) Date of filing: 09.06.2011
(51) Int. Cl.: C07D 405/04, C07D 401/04, C07D 211/78, C07D 213/62, C07D 213/61

(54) **PROCESS FOR PREPARATION OF 2, 3-DIARYL-5-SUBSTITUTED PYRIDINES AND THEIR INTERMEDIATES**
VERFAHREN ZUR HERSTELLUNG VON 2,3-DIARYL-5-SUBSTITUIERTEN PYRIDINEN UND DEREN ZWISCHENSTOFFEN
PROCÉDÉ DE PRÉPARATION DE PYRIDINES À SUBSTITUTION 2,3-DIARYL-5 ET LEURS INTERMÉDIAIRES

(30) Priority: 16.06.2010 IN 1812MU2010
(43) Date of publication of application: 24.04.2013
(73) Proprietor: Glenmark Generics Limited, Navimumbai 400709 (IN)
(72) Inventor: DSOUZA, Francis, Paul, Maharastra 400703 (IN); CRASTO, Anthony, Melvin, Maharashtra 422601 (IN); GHARPURE, Milind, Maharashtra 411038 (IN); NAYKODI, Sachin Bhagwan, Maharashtra 400705 (IN)
(74) Representative: HGF Limited
(86) International application number: PCT/IN2011/000388
(87) International publication number: WO 2011/158250

(56) References cited:
- EP-A2- 1 023 266
- WO-A1-98/47871
- WO-A1-2005/085199
- US-A- 6 040 319
- US-A- 6 040 319
- US-A- 6 040 450
- DAVIES I W ET AL: "A PRACTICAL SYNTHESIS OF A COX-2-SPECIFIC INHIBITOR", JOURNAL OF ORGANIC CHEMISTRY, ACS, US, vol. 65, 1 January 2000 (2000-01-01), pages 8415-8420, XP002326845, ISSN: 0022-3263, DOI: 10.1021/JO000870Z
- MARCOUX JEAN-FRANCOIS ET AL: "Annulation of Ketones with Vinamidinium Hexafluorophosphate Salts: An Efficient Preparation of Trisubstituted Pyridines", ORGANIC LETTERS, AMERICAN CHEMICAL SOCIETY, US, vol. 2, no. 15, 1 January 2000 (2000-01-01), pages 2339-2341, XP002199239, ISSN: 1523-7060, DOI: 10.1021/OL006097B

## Description

### FIELD OF THE INVENTION

The present invention relates to a process for the preparation of 2,3-diaryl-5-substituted pyridines, compounds of formula I. The present invention also relates to novel compounds of formula II, and a process for the preparation thereof, wherein compounds of formula II may be used as intermediates for the preparation of 2,3-diaryl-5-substituted pyridines.

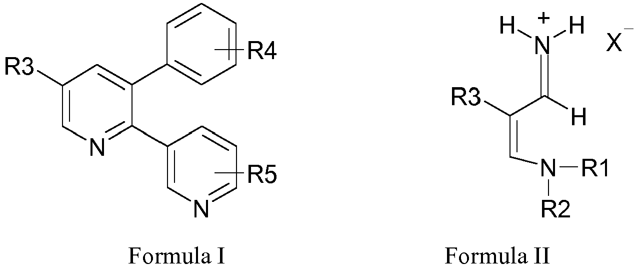

More specifically, the present invention relates to a process for synthesizing 5-chloro-3-[4-methylsulfonyl)phenyl]-2-(2-methyl-5-pyridinyl)pyridine, a compound of formula A. Formula A is commonly known as etoricoxib, which is a selective cyclooxygenase-2 inhibitor(COX-2).

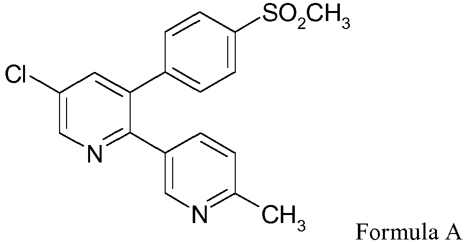

### BACKGROUND OF THE INVENTION

Selective inhibitors of cyclooxygenase-2 are a sub-class of drugs known as non-steroidal anti-inflammatory drugs (NSAIDs). Conventional NSAIDs block both forms of the cyclooxygenase enzyme and although active in reducing pain and swelling associated with the inflammatory process can produce severe side effects. The identification of the COX-2 enzyme associated with inflammation has provided a viable target of inhibition which more effectively reduces inflammation and produces fewer and less drastic side effects.

Etoricoxib is a potent and selective COX-2 inhibitor, which is effective in the management of chronic pain in rheumatoid arthritis, osteoarthritis and other COX-2 mediated disorders. Etoricoxib is designated chemically as 5-chloro-3-[4-methylsulfonyl) phenyl]-2-(2-methyl-5-pyridinyl)pyridine and is represented by the compound of formula A.

WO 2005/085199 discloses crystalline forms of Etoricoxib. Davies, I. W., et al., "A practical synthesis of a cox-2-specific inhibitor", Journal of Organic Chemistry, 2000, vol. 65, pages 8415-8420 discloses a number of synthetic strategies to the Cox-2 specific inhibitor. Marcoux, J-F., et al., "Annulation of ketones with vinamidinium hexafluorophosphate salts: An efficient preparation of trisubstituted pyridines", Organic Letters, 2000, vol. 2, no. 15, pages 2339-2341 discloses an α-aryl ketone reaction with vinamidinium hexafluorophosphate salts to give access to the corresponding 3-arylpyridines.

United States Patent No. 6040319 (US'319) discloses a process for preparing a series of 2-pyridyl-3-(4-methylsulfonyl)phenylpyridines. US '319 discloses a process for preparing these compounds by reacting ketosulfone with the vinamidinium salt under basic conditions. This was followed by treatment with ammonia to obtain the pyridine ring. This patent exemplifies the preparation of etoricoxib by reacting 2-chloro-N,N-dimethylamino trimethinium hexafluorophosphate with ketosulfone in the presence of equimolar amount of tertiary butoxide in tetrahydrofuran and quenching the resultant adduct in a mixture of acetic acid and trifluoroacetic acid. Ring closure of the pyridine ring occurred upon heating at reflux in the presence of aqueous ammonium hydroxide

There is need in the art for a process for preparing compounds of formula I having sufficient purity and yield to meet quality and regulatory standards for pre-clinical and commercial use. The present invention presents a novel process for preparing compounds of formula I via a novel intermediate compound of formula II. The present invention also provides a process for improving the colour of the final product thereby removing coloured impurities. The ensuing product from the present invention herein described, conforms to ICH grade purity specifications for bulk drugs, which requires that known impurity should not be more than 0.15% and unknown impurity should not be more than 0.1%.

### SUMMARY OF THE INVENTION

The present invention provides a process for preparing a compound of formula I: 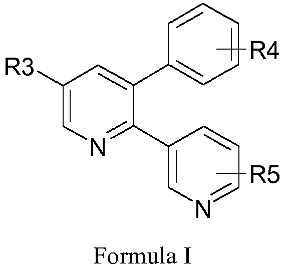 wherein R3 is halo; R4 and R5 are independently selected from the group consisting of C₁₋₆alkyl, halo-, C₁₋₄alkoxy, C₁₋₄alkythio, nitro-, amino-, C₁₋₆alkylamino, di-C₁₋₆alkylamino, cyano, -S(O)ₙH,- C₁₋₆alkylsulfonyl, arylsulfonyl, -S(O)ₙNH₂ and - S(O)ₙNHC₁₋₆ alkyl, wherein the alkyl and aryl groups of the substituent of R4 and R5 may be further substituted with alkyl, aryl, halo, hydroxyl, aryloxy;
and n is 0,1 or 2,
the process comprising reacting a compound of formula II : 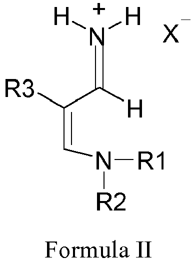 wherein R1 and R2 are independently selected from the group consisting of hydrogen, C₁-C₆ alkyl, aryl, -arylalkyl and -alkylaryl ; R3 is halo and X⁻ is a negatively charged counterion,
with a compound of formula III.: 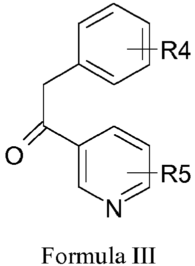 wherein R4 and R5 are as previously defined.

The present invention provides a compound of formula II 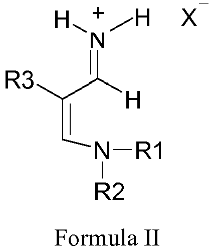 wherein R1 and R2 are independently selected from the group consisting of hydrogen, C₁-C₆ alkyl, aryl, -arylalkyl and -alkylaryl ; R3 is halo and X⁻ is a negatively charged counterion.

The present invention provides 2 chloro-3-(dimethylamino) prop-2-en-1-iminium chloride

The present invention provides a process for preparing compounds of formula II, wherein R1, R2 and R3 are as previously defined and X⁻ is halide, the process comprising reacting a compound of formula V, 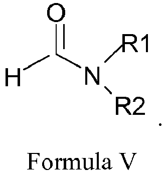 wherein R1 and R2 are independently selected from the group consisting of hydrogen, C₁-C₆ alkyl, aryl, -arylalkyl and-alkylaryl;
with oxalyl chloride, haloacetyl chloride and ammonia.

The present invention provides the process of preparation of etoricoxib, compound of formula A, the process comprising the use of the novel compounds of formula II, as intermediates.

The present invention provides etoricoxib having less than 0.1% of compound of formula IV, as measured by high performance liquid chromatography

The present invention provides pharmaceutical compositions of etoricoxib, obtained as herein described, and at least one pharmaceutical carrier.

### BRIEF DESCRIPTION OF THE DRAWINGS

Fig 1: is ¹³C NMR of 2 chloro-3-(dimethylamino) prop-2-en-1-iminium chloride salt, compound of formula II.
Fig 2: is DSC of2 chloro-3-(dimethylamino) prop-2-en-1-iminium chloride salt, compound of formula II.
Fig 3: is X-ray powder diffractogram of etoricoxib obtained as per Example 6.
Fig 4: is HPLC chromatogram for etoricoxib as per Example 6

### DETAILED DESCRIPTION OF THE INVENTION

The present invention relates to a process for the preparation of 2,3-diaryl-5-substituted pyridines, compounds of formula I. The present invention also relates to novel compounds of formula II, and a process for the preparation thereof, wherein compounds of formula II may be used as intermediates for the preparation of 2,3-diaryl-5-substituted pyridines.

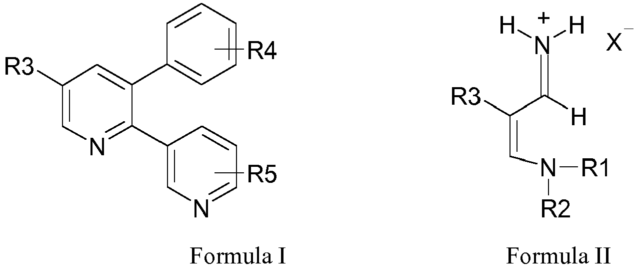

More specifically, the present invention relates to a process for synthesizing 5-chloro-3-[4-methylsulfonyl)phenyl]-2-(2-methyl-5-pyridinyl)pyridine, a compound of formula A. Formula A is commonly known as etoricoxib, which is a selective cyclooxygenase-2 inhibitor(COX-2).

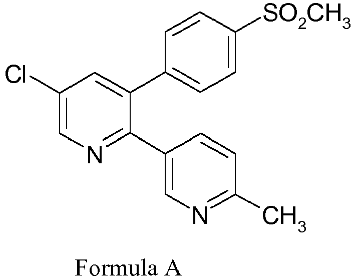

The present invention provides a process for the preparation of a compound of formula I: 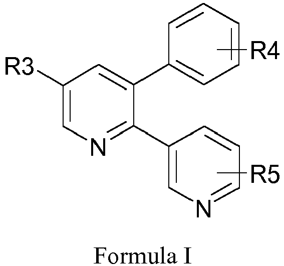 wherein R3 is selected from the group consisting of C₁₋₆alkyl, C₆₋₁₀ aryl, alkylaryl, halo, nitro, amino, C₁₋₆alkylamino, di-C₁₋₆alkylamino, cyano, -S(O)ₙH, C₁₋₆alkylsulfonyl,arylsulfonyl,-S(O)ₙNH₂ and -S(O)ₙNHC₁₋₆ alkyl; R4 and R5 are independently selected from the group consisting of C₁-₆alkyl, halo, C₁₋₄alkoxy, C₁₋₄alkythio, nitro, amino, C₁₋₆alkylamino, di-C₁₋₆alkylamino, cyano, S(O)ₙH,-C₁₋₆alkylsulfonyl,arylsulfonyl,-S(O)ₙNH₂ and -S(O)ₙNHC₁₋₆ alkyl, wherein the alkyl and aryl groups of the substituent of R3, R4 and R5 may be further substituted with alkyl, aryl, halo, hydroxyl, aryloxy
and n is 0,1 or 2,
the process comprising reacting a compound of formula II 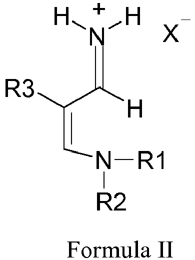 wherein R1 and R2 are independently selected from the group consisting of hydrogen, C₁-C₆ alkyl, aryl, -arylalkyl and -alkylaryl ; R3 is as defined above and X⁻ is a negatively charged counterion,
with a compound of formula III. 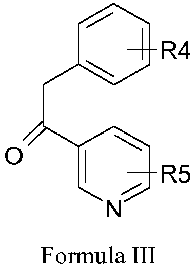 wherein R4 and R5 are as previously defined.

The term "alkyl" as used herein includes a straight or branched chain hydrocarbon containing from 1 to 6 carbon atoms. Representative examples of alkyl include, but are not limited to, methyl, ethyl, n-propyl, isopropyl, n-butyl, sec-butyl, tert-butyl, n-pentyl, isopentyl, neopentyl, n-hexyl.

The term "aryl" as used herein, refers to aromatic ring systems, which may include fused rings. Representative examples of aryl include, but are not limited to, phenyl, and naphthyl, anthracenyl, phenanthrenyl.

The term "alkylaryl" as used herein, refers to an aryl group, as defined herein, appended to the parent molecular moiety through an alkyl group, as defined herein. Representative examples of alkylaryl include, but are not limited to, benzyl, 2-phenylethyl, 3-phenylpropyl, and 2-naphth-2-ylethyl.

The term "arylalkyl" as used herein, refers to an aryl group, as defined herein, appended to the parent molecular moiety and substituted with an alkyl group, as defined herein. Representative examples of arylalkyl include, but are not limited to tolyl and phenyl ethyl.

The term "alkylamino" as used herein, refers to an amino group monosubstituted with a lower alkyl group, as defined herein, and appended to the parent molecular moiety through a nitrogen atom. Representative examples of alkylamino include, but are not limited to, methylamino, ethylamino, propylamino, isopropylamino, butylamino, sec-butylamino and tert-butylamino.

The term "dialkylamino" as used herein, refers to an amino group disubstituted with identical or different lower alkyl groups as defined herein, and appended to the parent molecular moiety through a nitrogen atom. Representative examples of dialkylamino include, but are not limited to, dimethylamino, diethylamino, dipropylamino, methylpropylamino and diisopropylamino.

The term "alkylsulfonyl" as used herein, means an alkyl group, as defined herein, appended to the parent molecular moiety through a sulfonyl group, as defined herein. Representative examples of alkylsulfonyl include, but are not limited to, methylsulfonyl and ethylsulfonyl.

The term "arylsulfonyl" as used herein, means an aryl group, as defined herein, appended to the parent molecular moiety through a sulfonyl group. Representative examples of arylsulfonyl include, but are not limited to benzenesulfonyl and p-toluene sulfonyl.

The term "aryloxy" as used refers to an aryl group, as defined herein, appended to the parent molecular moiety through an oxygen atom. Representative examples of aryloxy include, but are not limited to, phenoxy, naphthyloxy.

In one embodiment, the present invention provides a process for the preparation of a compound of formula I: 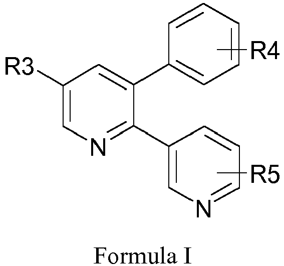 wherein R3 is halo; R4 and R5 are independently selected from the group consisting of C₁₋₆alkyl, halo, C₁₋₄alkoxy, C₁₋₄alkythio, nitro, amino, C₁₋₆alkylamino, di-C₁₋₆alkylamino, cyano, S(O)ₙH,- C₁₋₆alkylsulfonyl,arylsulfonyl,-S(O)ₙNH₂ and -S(O)ₙNHC₁₋₆ alkyl, wherein the alkyl and aryl groups of the substituent of R4 and R5 may be further substituted with alkyl, aryl, halo, hydroxyl, aryloxy
and n is 0,1 or 2,
the process comprising reacting a compound of formula II 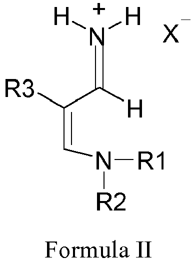 wherein R1 and R2 are independently selected from the group consisting of hydrogen, C₁-C₆ alkyl, aryl, -arylalkyl and -alkylaryl ; R3 is halo and X⁻ is a negatively charged counterion;
with a compound of formula III. 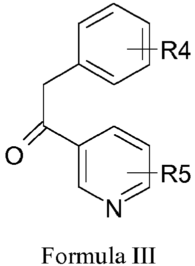 wherein R4 and R5 are as previously defined.

The reaction of compound of formula II with compound of formula III may be carried out in acidic medium.

In one preferred embodiment, the present invention provides a process for the purification of compound of formula I, the process comprising dissolving the compound of formula I in tetrahydrofuran, where the tetrahydrofuran used is an amount that is sufficient to solubilize the compound of formula I; heating the tetrahydrofuran solution of compound of formula I at about 45°C to about 50°C; and maintaining at about that temperature; adding water to the solution, wherein, preferably the water is added slowly to the tetrahydrofuran solution, and stirring the mixture. Upon completion of addition, preferably cooling the mixture to about room temperature to precipitate the solid. The solid obtained is isolated by filtration. Optionally, this purification process may be repeated.

In one embodiment, the present invention provides a recrystallization process for the compound of formula I, obtained by the process described herein, the process comprising
(a) adding isopropyl alcohol to compound of formula I to obtain a mixture;
(b) heating the mixture; and
(c) cooling the mixture to obtain compound of formula I.

The mixture may be heated in the temperature range of about 55°C to about 80°C. Preferably, the temperature is in the range of about 65°C to about 70°C. , Optionally, the mixture may be filtered. The filtrate thus obtained is cooled to about room temperature or about lower than room temperature and the obtained compound of formula I is isolated.

The present invention provides a process for the preparation of a compound of formula IA 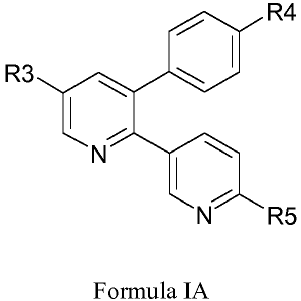 wherein R3 is halo and R4 is selected from the group consisting of S(O)ₙH, C₁₋₆alkylsulfonyl,arylsulfonyl,-S(O)ₙNH₂ and -S(O)ₙNHC₁₋₆ alkyl, and R5 is selected from the group consisting of C₁₋₆alkyl, halo, C₁₋₄alkoxy and C₁₋₆alkyl; the process comprising reacting a compound of formula II, wherein R1 and R2 are both independently methyl, R3 is halo and X⁻ is a negatively charged counterion, with a compound of formula III A wherein R4 and R5 are as defined above.

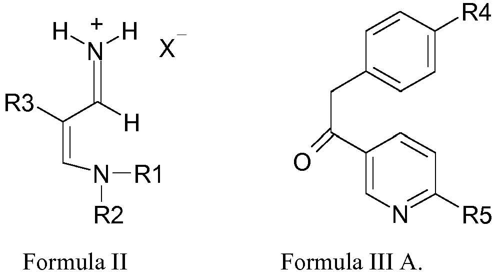

The present invention provides a process for the preparation of a compound of formula IA wherein R3 is halo and R4 is C₁₋₆alkylsulfonyl and R5 is C₁₋₆alkyl, the process comprising
reacting a compound of formula II, wherein R1 and R2 are both independently methyl, R3 is halo and X⁻ is a negatively charged counterion, with a compound of formula III A wherein R4 is C₁₋₆alkylsulfonyl and R5 is C₁₋₆alkyl.

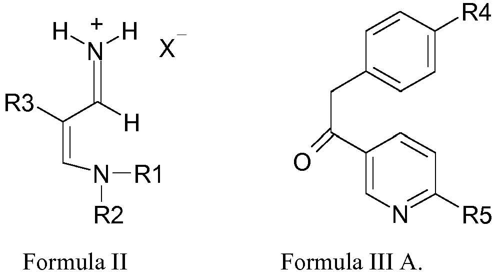

The present invention provides a process for the preparation of a compound of formula A, (compound of formula IA wherein R3 is chloro and R4 is methylsulfonyl and R5 is methyl) 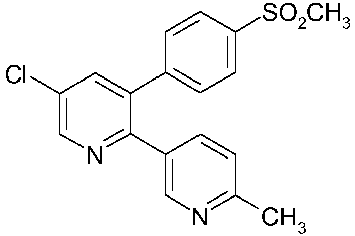 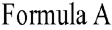 the process comprising reacting a compound of formula II, wherein R1 and R2 are both independently methyl, R3 is chloro and X⁻ is selected from chloride and hexafluorophosphate, with a compound of formula IIIA wherein R4 is methylsulfonyl and R5 is methyl.

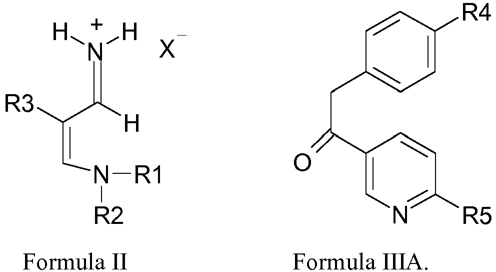

The reaction of compound of formula II with compound of formula IIIA may be carried out in acidic medium.

The acidic medium for the reaction may be selected from acetic acid, propionic acid, trifluoroacetic acid, sodium acetate and acetic acid, potassium acetate and acetic acid, zinc acetate and acetic acid, ammonium acetate and acetic acid and citric acid and sodium citrate and the like. Preferably the reaction is carried out in presence of sodium acetate and acetic acid. The pH of the acidic medium is preferably in the range of about 4 to 5.

The reaction may be carried out in the presence or absence of solvent. In one preferred embodiment, the reaction is carried out in absence of the solvent.

The reaction may be carried out at a temperature in the range of about 60 °C to about 120 °C. The reaction is carried out for a period of about 18 hours to about 30 hours. Preferably the reaction is carried out by heating the reaction mass in the absence of a solvent in the temperature range of about 90°C to about 100°C for a period of about 18 hours to about 24 hours.

In one embodiment, the present invention provides a process for the preparation of a compound of formula A, the process comprising reacting a compound of formula II, wherein R1 and R2 are both independently methyl, R3 is chloro and X⁻ is chloride, with a compound of formula IIIA wherein R4 is methylsulfonyl and R5 is methyl, in the presence of sodium acetate and acetic acid. Upon completion, the reaction mass is basified.

In one preferred embodiment, the present invention provides a process for the preparation of a compound of formula A, the process as described above, wherein an organic solvent is added to the reaction mass prior to basification. The organic solvent selected for basification may be selected from aromatic hydrocarbon and aliphatic hydrocarbon. Preferably an aromatic hydrocarbon like toluene is added to the cooled reaction mass before adding a base. The base may be selected from an organic base or inorganic base. The inorganic base may be selected from the carbonates or bicarbonates of alkali metals, alkaline earth metal and the like; for example, sodium carbonate, potassium carbonate, sodium bicarbonate, potassium bicarbonate and the like. The organic base may be selected from the group consisting of triethylamine, diethylamine and the like. Preferably the pH is in the range of about 8-9 and is adjusted by using sodium carbonate. After basification the organic layer is separated and is worked up by standard procedures to obtain the compound of formula I.

In one embodiment, the present invention provides a process for the preparation of a compound of formula A, the process as described above, wherein the organic layer obtained after basification is treated with sodium dithionate solution. Preferably the organic layer containing the compound of formula I is subjected to washing with sodium dithionate solution and the organic layer obtained after washing with sodium dithionate is worked up by standard procedures.

The present invention provides a process for the purification of the compound of formula A (etoricoxib), the process comprising treating the compound of formula A with sodium dithionate. In one embodiment, the present invention provides a process for the purification of the compound of formula A (etoricoxib), the purification process comprising
- dissolving the compound of formula A in an organic solvent selected from aromatic hydrocarbon and aliphatic hydrocarbon. Preferably an aromatic hydrocarbon like toluene is used.
- washing the solution of compound of formula A (etoricoxib) in toluene with aqueous sodium dithionate solution;
- isolating the compound of formula A from the toluene layer by conventional means known in the art, for example by means of rotaevaporation.

The present invention provides a process for the purification of compound of formula A, the process comprising a solvent/anti-solvent purification technique. In one embodiment, the present invention provides a process for the purification of the compound of formula A, the process comprising
(a) dissolving compound of formula A in tetrahydrofuran to form a solution ;
(b) adding water to the above solution ; and
(c) isolating the compound of formula A from the solution.

In one preferred embodiment, the present invention provides a process for the purification of compound of formula A, the process comprising dissolving the compound of formula A in tetrahydrofuran, where the tetrahydrofuran used is an amount that is sufficient to solubilize the compound of formula I; heating the tetrahydrofuran solution of compound of formula I at about 45°C to about 50°C; and maintaining at about that temperature; adding water to the solution, wherein, preferably the water is added slowly to the tetrahydrofuran solution, and stirring the mixture. Upon completion of addition, preferably cooling the mixture to about room temperature to precipitate the solid. The solid obtained is isolated by filtration. Optionally, this purification process may be repeated.

In one embodiment, the present invention provides a process for purification of compound of formula A, the process comprising subjecting the compound of formula A to an acid-base treatment.

The present invention provides a process for purification of compound of formula A, the process comprising dissolving the compound of formula A in an organic solvent and adding an acid to the solution; separating the aqueous layer; basifying to precipitate the compound of formula A. The acid may be selected from the group consisting of hydrochloric acid, sulfuric acid, nitric acid and the like The base may be selected from the group consisting of alkali or alkaline metal carbonate, alkali or alkaline metal bicarbonates, ammonia, organic bases such as triethylamine and the like.

In one embodiment, the present invention provides a process for the purification of compound of formula A, the process comprising dissolving the compound of formula A in toluene and adding hydrochloric acid; separating the aqueous layer ; basifying with aqueous ammonia to precipitate the product; and filtering the product.

The present invention provides a recrystallization process for the compound of formula A, obtained by the process described herein, the process comprising crystallizing the compound of formula A in a solvent system. The solvent system includes an isopropyl alcohol or mixture of isopropyl acetate and hexane.

In one embodiment, the present invention provides a recrystallization process for the compound of formula A (etoricoxib), obtained by the process described herein, the process comprising
a. adding isopropyl alcohol to etoricoxib to obtain a mixture;
b. heating the mixture; and
c. cooling the mixture to obtain etoricoxib.

The mixture may be heated in the temperature range of 55-80°C. Preferably the temperature is in the range of 65-70°C. If required the mixture was filtered. The filtrate thus obtained was cooled to room temperature or below and the obtained compound of formula I was isolated.

The mixture may be cooled with continuous stirring or with occasional stirring. Occasional stirring, as used herein, is intended to mean a intermittent stirring. i.e. not continuous, and which may be carried out once in about 20 minutes to about 40 minutes. The cooling may be carried out for a period of about 1 hour.

In one embodiment the present invention provides a process for controlling the particle size of compound of formula A (etoricoxib) depending on the rate of stirring during cooling of the reaction mixture.

In one embodiment, when the mixture in the process directly described above, is stirred continuously, etoricoxib with D₉₀ less than about 15 µm is obtained.

In one embodiment of the present invention, the etoricoxib has D₁₀ of about 1.32µm, D₅₀ about 4.12 µm, and D₉₀ about 10.19 µm

In one embodiment when the mixture is stirred once in about 30 minutes the D₉₀ more than about 40µm is obtained.

In one embodiment of the present invention, the D90 of etoricoxib obtained is about 55µm.

In one embodiment of the present invention, the etoricoxib has D₁₀ about 2.98 µm, D₅₀ about 12.65 µm, and D₉₀ about 55.94µm

In one embodiment, the present invention provides a process for the purification of etoricoxib, compound of formula A, the process comprising
a. washing the etoricoxib, with aqueous sodium dithionate solution;
b. dissolving the etoricoxib in tetrahydrofuran ;
c. precipitating the etoricoxib by addition of water;
d. recrystallizing the etoricoxib from isopropyl alcohol; and
e. optionally subjecting the etoricoxib to acid base treatment.

The present invention provides etoricoxib having a color index of less than 0.2 AU as measured by colorimetry.

In one embodiment present invention provides etoricoxib having a color index of about 0.1 AU as measured by Perkin Lambda 35 UV/VIS Spectrophotometer

The color index is measured by Perkin Lambda 35 UV/VIS Spectrophotometer

The absorbance of 5%w/v solution in methanol at 420nm was measured. 2.5g of sample was taken into a 50ml volumetric flask. The sample was dissolved in methanol and diluted up to the mark with methanol. The absorbance of test solution prepared was measured at 420nm in a 5 cm cell using methanol as a blank.

In one embodiment, the present invention provides a process for preparing etoricoxib having a color index of less than 0.2 AU, the process comprising
a. washing the etoricoxib, obtained by the processes herein described, dissolved in toluene with aqueous sodium dithionate solution;
b. separating the toluene layer from the aqueous layer;
c. removing the toluene layer to obtain etoricoxib;
a. dissolving the etoricoxib in tetrahydrofuran ;
b. precipitating the etoricoxib by addition of water;
c. recrystallizing the etoricoxib from isopropyl alcohol;
d. optionally subjecting the etoricoxib to acid base treatment

In one embodiment, the present invention provides a process for the preparation of etoricoxib having a color index of less than 0.2 AU, the process comprising an acid base treatment process comprising dissolving the etoricoxib in an organic solvent; adding an acid; separating the aqueous layer; then basifying to precipitate the compound of formula A; optionally filtering the product.

The present invention provides a process for the preparation of etoricoxib having a color index of less than 0.2 AU, the process comprising an acid base treatment process comprising dissolving the etoricoxib in toluene and adding hydrochloric acid; separating the aqueous layer basifying with aqueous ammonia to precipitate the product; and filtering the product.

In one aspect, the present invention presents etoricoxib, obtained by the process herein described that meets International Conference on Harmonization of Technical Requirements for Registration of Pharmaceuticals for Human Use (ICH) grade purity specifications.

The present invention provides etoricoxib having a chemical purity of greater than about 99.60%, as measured by high performance liquid chromatography.

The present invention provides etoricoxib having less than about 1 % of total impurities as measured by high performance liquid chromatography (HPLC).

Specifically the present invention provides etoricoxib, having less than about 0.1% of compound of formula IV as measured by high performance liquid chromatography. Compound of formula IV is designated as furan impurity.

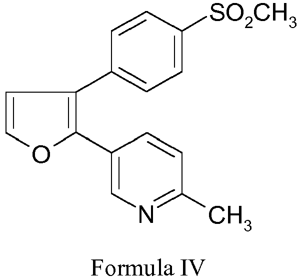

More specifically, the present invention provides etoricoxib, having less than about 0.05% of compound of formula IV, as measured by high performance liquid chromatography.

The present invention provides etoricoxib, obtained by the process disclosed herein, having less than about 2000 parts per million (ppm) isopropanol, less than about 100 ppm of acetone, less than about 100ppm tetrahydrofuran as measured by gas chromatography.

In another aspect, the present invention provides a compound of formula II. 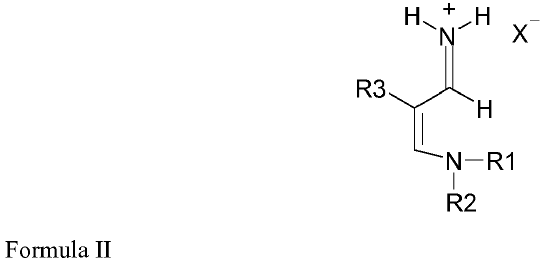 wherein R1 and R2 are independently selected from the group consisting of hydrogen, C₁₋C₆ alkyl, aryl, -arylalkyl and -alkylaryl ; R3 is selected from the group consisting of C₁₋₆alkyl, C₆₋₁₀ aryl, alkylaryl, halo, nitro, amino, C₁₋₆alkylamino, di-C₁₋₆alkylamino, cyano, S(O)ₙH, C₁₋₆alkylsulfonyl,arylsulfonyl,-S(O)ₙNH₂ and -S(O)ₙNHC₁₋₆ alkyl and X⁻ is a negatively charged counterion.

The negatively charged counterion X⁻ is selected from the group consisting of halides, sulfonates, HSO₄⁻ , SO₄²⁻, HCO₃⁻, CO₃²⁻, borate, trifluoroacetate, perchlorate, phosphate, acetate, lactate, succinate, propionate, oxalate, butyrate, ascorbate, citrate, dihydrogen citrate, tartrate, taurocholate, glycocholate, cholate, hydrogen citrate, maleate, benzoate and folate.

Preferred values of X⁻ are halides like chloride, bromide, iodide, fluoride and phosphates such as hexafluorophosphate and the like.

In one embodiment, the present invention provides compound of formula II 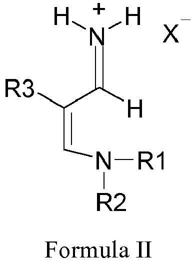 wherein R1 and R2 are independently selected from the group consisting of hydrogen, C₁-C₆ alkyl, aryl, -arylalkyl and -alkylaryl; R3 is halo and X ⁻ is a negatively charged counterion.

R1 and R2 are preferably C₁-C₆ alkyl, R3 is halo; while X⁻ is selected from halide and phosphate. More preferably the present invention provides compounds of formula II, wherein R1 and R2 are both independently methyl, R3 is chloro and X⁻ is selected from chloride and hexafluorophosphate.

In one embodiment, the present invention provides a compound of formula II, wherein R1 and R2 are both independently methyl, R3 is chloro and X⁻ is chloride, 2 chloro-3-(dimethylamino) prop-2-en-1-iminium chloride.

In one embodiment, the present invention provides 2 chloro-3-(dimethylamino) prop-2-en-1-iminium chloride that exhibits a ¹³C NMR spectrum with signals at about 34.30, 48.8, 94.95, 158.68, 161.08 ppm.

¹³C NMR spectrum was recorded in DMSO d₆ using 100 MHz Bruker 400 NMR spectrometer.

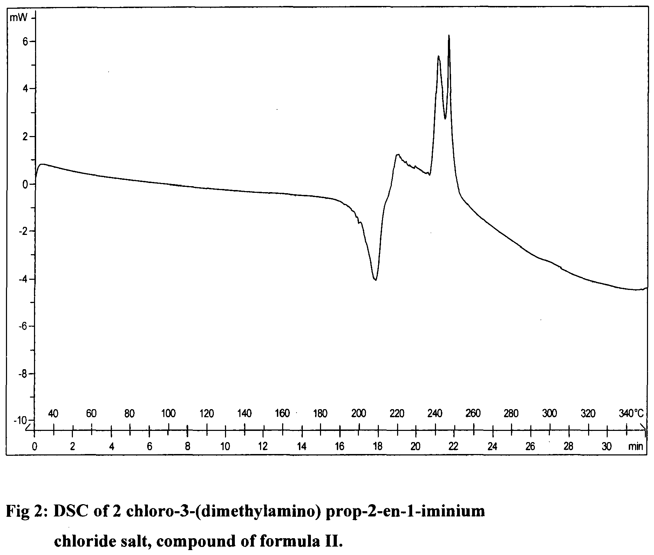

In one embodiment, the present invention provides 2-chloro-3-(dimethylamino) prop-2-en-1-iminiumchloride that exhibits differential scanning calorimetry (DSC) endotherm curve, which is substantially in accordance with Fig. 2.

Approximately 1-5mg of sample was accurately weighed into an aluminum DSC pan with lid. The sample was placed then into a Mettler Toledo DSC822^{e} equipped with a liquid nitrogen cooling unit and allowed to equilibrate at 30°C until stable heat flow response was seen. A dry nitrogen purge gas at a flow rate of 50ml/min was used to produce the inert atmosphere and prevent oxidation of the sample during heating. The sample was scanned from 50-250°C at rate of 10°C/min and resulting heat flow response was measured against temperature.

In another aspect, the present invention provides a process for preparing novel compounds of formula II, wherein X⁻ is halide, the process comprising reacting a compound of formula V, 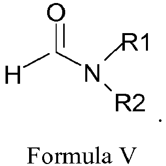 ; wherein R1 and R2 are independently selected from the group consisting of hydrogen, C₁-C₆ alkyl, aryl, -arylalkyl and -alkylaryl, with oxalyl chloride, haloacetyl chloride ammonia.

The haloacetyl chloride may be selected from chloroacetyl chloride, bromoacetyl chloride and the like. Preferably, chloroacetyl chloride is used.

In one preferred embodiment, the present invention provides a process for preparing novel compounds of formula II, the process comprising
- reacting the compound of formula V with oxalyl chloride. The oxalyl chloride is added very slowly to the compound of formula V, preferably dropwise and the reaction mass is stirred. The reaction mass is maintained in the temperature range of about 0 °C to about 10 °C for a period of about 1 hour to about 2 hours.
- slowly adding haloacetyl chloride slowly to the reaction mass
- heating the reaction mass to the temperature range of about 70 °C to about 75°C.

The reaction mass is maintained in the temperature range of about 70 °C to about 75 °C for a period of about 3 hours to about 4 hours.
- cooling the reaction mass preferably to room temperature
- adding an alcohol to the cooled reaction mass. The alcohol may be selected from methanol, ethanol, isopropanol and the like. Preferably methanol is added to the cooled reaction mass.
- adding an Aqueous ammonia to the alcoholic reaction mixture, in an amount of aqueous ammonia that the pH of the obtained reaction mixture is in the range of 8-9.
- stirring the reaction mixture at room temperature
- separating the solids
- isolating the compound of formula II from the filtrate. Preferably the compound of formula II is isolated by distilling out the filtrate and stripping the obtained mass with alcohol, followed by addition of alcohol and aqueous ammonia to a pH in the range of 8-9 and filtering out the resultant solid.

The present invention provides the compound of formula II, in the process, as herein described, wherein X⁻ is halide may be converted to any other desired counterion. Illustratively, the hexafluorophosphate salt of compound of formula II may be obtained by reacting compound of formula II, wherein X⁻ is halide, with sodium hexafluorophosphate under acidic conditions or with hexafluorophosphoric acid.

The present invention presents a process for the preparation of a compound of formula II, wherein R1 and R2 are both independently methyl, R3 is chloro and wherein X⁻ is chloride, the process comprising reacting dimethylformamide, chloroacetyl chloride and oxalyl chloride; cooling the reaction mass preferably to about room temperature; adding an alcohol, preferably to the cooled reaction mass; adding aqueous ammonia to the alcoholic reaction mixture. The alcohol may be selected from methanol, ethanol, isopropanol and the like. The amount of aqueous ammonia used is such that the pH of the obtained reaction mixture is in the range of about 8-9. The reaction mixture is stirred at room temperature and the solids are separated. The ensuing compound of formula II, wherein R1 and R2 are both independently methyl, R3 is chloro and wherein X⁻ is chloride, is then isolated from the filtrate. Preferably the compound of formula II is isolated by distilling out the filtrate and stripping the obtained mass with alcohol, adding alcohol then aqueous ammonia to pH in the range of about 8-9 and filtering out the resultant solid. Optionally, the resultant solid is washed with isopropyl alcohol.

The present invention presents a purification process of a compound of formula II, obtained as herein described, wherein R1 and R2 are both independently methyl, R3 is chloro and wherein X⁻ is chloride, the process comprising recrystallizing the compound of formula II from acetone.

The present invention provides a pharmaceutical composition comprising etoricoxib, prepared by processes herein described above, and at least a pharmaceutically acceptable carrier. The pharmaceutical compositions may be administered to a mammalian patient in any dosage form, e.g., liquid, powder, elixir, injectable solution, etc. Dosage forms may be adapted for administration to the patient by oral, buccal, parenteral, ophthalmic, rectal and transdermal routes. Oral dosage forms include, but are not limited to, tablets, pills, capsules, troches, sachets, suspensions, powders, lozenges, elixirs and the like.

The following examples are provided to enable one skilled in the art to practice the invention and are merely illustrative of the invention. The examples should not be read as limiting the scope of the invention as defined in the features and advantages.

### EXAMPLES

### Example-1

### Preparation of 2 chloro-3-(dimethylamino) prop-2-en-1-iminium chloride salt, compound of formula II, wherein R1 and R2 are methyl and R3 is chlorine.

In a 2.0L 4-neck round bottom flask equipped with an overhead stirrer , double surface condenser, thermowell pocket placed in the cooling water bath, 373.45gm dimethyl formamide was added and cooled to about 0°C to about 10°C and 112.38gm oxalyl chloride was added slowly through the addition funnel. Once the addition was completed, the reaction mixture was maintained for about 2 hrs at about 0°C to about 10°C and 100.0gm chloroacetyl chloride was added slowly through the addition funnel. After the addition was over the reaction mass was brought to about 25°C to about 30°C and heated slowly to about 70°C to about 75°C and maintained for 3 hours at about 70°C to about 75°C and monitored the reaction by GC, until the chloroacetyl chloride was not more than about 5.0%.The reaction mass was cooled to about 25°C to about 30°C and 300ml(3volume) methanol was added and further cooled to about 0°C and the reaction mass was basified to pH 8-9 by 150ml aqueous NH₃. The separated solids were filtered and the clear filtrate was concentrated under vacuum at about 70°C to about 90°C, the reaction mass was stripped out with 200ml methanol (2vol) X 2 times or 200ml of isopropanol. The reaction mass was degassed for 30 min at about 80°C to about 90°C and cooled to about 55°C to about 60°C and diluted with 100ml(1vol) methanol or 200ml of isopropanol. Further the reaction mass was cooled to about 0°C to about 10°C and basified with 8-10ml aqueous NH₃, maintained for 1hour at this temperature and filtered, washed with isopropyl alcohol 200ml (2vol) X 2 times.

The wet product wt 157gm was dried in oven at about 50°C to about 55°C and dry wt: 131.0gm.

The dry product was purified by 1.3L (10vol) of acetone at about 52°C to about 55°C for 1hr and cooled to about 25°C to about 30°C and maintained for 1hr and filtered and washed with 130ml (1vol) of acetone and the wet wt obtained was 133gm.

### Optional purification

The above wet material was again purified with a mixture of 1.3L (10vol) of a mixture of 1:1 methylene chloride and acetone at about 42°C to about 45°C for 1hr and cooled to about 25°C to about 30°C and maintained for 1 hr and filtered and washed with 100 ml of 1:1 mixture of methylene chloride and acetone.

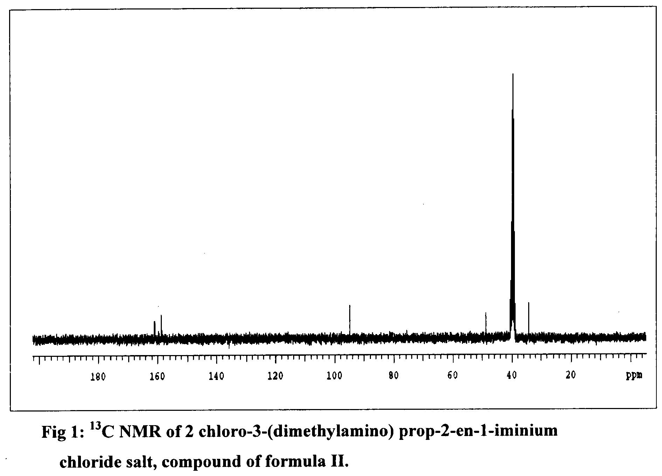

The wet material was dried in air oven for 6-8 hrs at about 55°C to about 60°C and dry wt:-128gm.
¹H-NMR (DMSOD6, δppm): 2.5, 3.26, 3.47, 8.12, 8.40
¹³C NMR (DMSOD6) ppm 34.30, 48.8, 94.95, 158.68, 161.08. Figure 1 is the ¹³C NMR. IR (KBr, cm"¹): 3065, 2031, 1909, 1643, 1577, 1490, 1355, 1333.
Mass spectra: Molecular Ion peak at 133.16; base peak at 104.16

### Example-2

### Preparation of 2 chloro-3-(dimethylamino) prop-2-en-1-iminium hexafluorophosphate salt, compound of formula II, wherein R1 and R2 are methyl and R3 is chlorine.

In a 250ml 4-neck round bottom flask equipped with overhead stirrer , thermowell pocket placed in water bath, added 10.0gm of (2E)-2 chloro-3-(dimethylamino) prop-2-en-1-iminium chloride salt and 25 ml purified water under stirring. A clear solution was obtained. The reaction mass was cooled to about 5°C to about 10°C and then 10ml hexafluorophosphoric acid was added slowly, while maintaining the temperature below about 10°C.

The reaction mass was maintained for 2hours below about 10°C and filtered the reaction mass to obtain 2 chloro-3-(dimethylamino) prop-2-en-1-iminium hexafluorophosphate salt wet wt: 12gm and dried in air oven to get dry wt: 8.8gm.

### Example-3

### Preparation of 5-chloro-3-[4-methylsulfonyl)phenyl]-2-(2-methyl-5-pyridinyl)pyridine, a compound of formula A, etoricoxib.

In a 500ml 4-neck round bottom flask equipped with overhead stirrer, double surface condenser, thermowell pocket, placed in an oil bath, 125ml glacial acetic acid was added followed by 50gm ketosulfone and 87.61gm (2E)-2 chloro-3-(dimethylamino) prop-2-en-1-iminium chloride salt and 99.22gm sodium acetate and the funnel was rinsed with 25 ml glacial acetic acid at about 25°C to about 30°C. The reaction mass was heated to about 95°C to about 100°C and maintained for 24 hrs and monitored by HPLC, until the ketosulfone was not more than 5.0%. The reaction mass was cooled to about 25°C to about 30°C and added 500ml (10volume) toluene and basified the reaction mass with 18-20% aqueous sodium carbonate solution. The aqueous layer was again extracted with hot toluene at about 45°C to about 50°C and the combined toluene layers was washed with brine solution followed by water wash to the organic layer. The organic layer was charcoalised with NORIT® charcoal and filtered on a hyflo bed and the organic layer was concentrated to obtain an oily residue. The solid was isolated by a mixture of tetrahydrofuran and water. The solid was further dissolved in tetrahydrofuran and precipitated by water to remove the furan impurity formed in the reaction mass and crude etoricoxib dry weight 33.0gm was isolated.

### Example-4

### Preparation of 5-chloro-3-[4-methylsulfonyl)phenyl]-2-(2-methyl-5-pyridinyl)pyridine, a compound of formula A, etoricoxib.

In a 1.0 L 4-neck round bottom flask equipped with overhead stirrer, double surface condenser, thermowell pocket placed in oil bath, 275ml glacial acetic acid was added followed by 100gm ketosulfone and 175.0gm (2E)-2 chloro-3-(dimethylamino) prop-2-en-1-iminium chloride salt and 198.0gm sodium acetate and the funnel was rinsed with 25 ml glacial acetic acid at about 25°C to about 30°C. The reaction mass was heated to about 95°C to about100°C and maintained for 24 hrs and monitored by HPLC until the ketosulfone was not more than about 5.0%. The reaction mass was cooled to about 25°C to about 30°C and 1.0L (10volume) toluene was added and basified the reaction mass with 18- 20% aqueous sodium carbonate solution.

The aqueous layer was again extracted with hot toluene at about 45°C to about 50°C and the toluene layers were combined and washed with brine solution followed by water wash. The organic layer was charcoalised with NORIT® charcoal and filtered on a hyflo bed and then concentrated under vacuum to obtain an oily residue. The crude etoricoxib was isolated by tetrahydrofuran and water mixture. The solids were further dissolved in tetrahydrofuran and precipitated by water to remove the furan impurity formed in the reaction mass and the crude etoricoxib dry weight 48.0gm was isolated.
HPLC purity: 99.03%
Furan impurity: 0.073%

### Example-5

### Preparation of 5-chloro-3-[4-methylsulfonyl)phenyl]-2-(2-methyl-5-pyridinyl)pyridine, a compound of formula A, etoricoxib.

In a 1.0 L 4-neck round bottom flask equipped with overhead stirrer, double surface condenser, thermowell pocket placed in an oil bath, 500ml glacial acetic acid was added followed by 250gm ketosulfone and 474.6gm (2E)-2 chloro-3-(dimethylamino) prop-2-en-1-iminium chloride salt and 496.2gm sodium acetate and the funnel was rinsed with 250 ml glacial acetic acid at about 25°C to about 30°C. The reaction mass was heated to about 95°C to about 100°C and maintained for 24 hrs and monitored by HPLC until the ketosulfone was not more than 5.0%. The reaction mass was cooled to about 25°C to about 30°C and toluene was added and the reaction mass was basified with 18- 20% aqueous sodium carbonate solution. The reaction mass was heated to about 45°C to about 50°C. The aqueous and organic layers were separated. The aqueous layer was again extracted with hot toluene and the toluene layers were combined and washed with 5 % sodium dithionate solution. The toluene layer was then again separated followed by washing it with water. The organic layer was charcoalised with NORIT® charcoal and filtered on a hyflo bed and the organic layer was concentrated under vacuum to obtain a residue. THF (tetrahydrofuran) was added to the residue and the reaction mass was heated. Purified water was slowly added to the reaction mass and the reaction mixture was cooled followed by stirring. The solids obtained were further dissolved in tetrahydrofuran and precipitated by addition of water to obtain compound of formula A. The compound of formula A obtained was recrystallized from isopropyl alcohol to obtain a solid which was dissolved in toluene and acidified with concentrated HCl. The aqueous layer was separated and basified with aqueous ammonia and pH was adjusted to 9. The solid obtained was filtered and dried.
HPLC purity: 99.77%
Furan impurity: Below detection limit

### Example-6

### Preparation of 5-chloro-3-[4-methylsulfonyl)phenyl]-2-(2-methyl-5-pyridinyl) pyridine, a compound of formula A. (Etoricoxib)

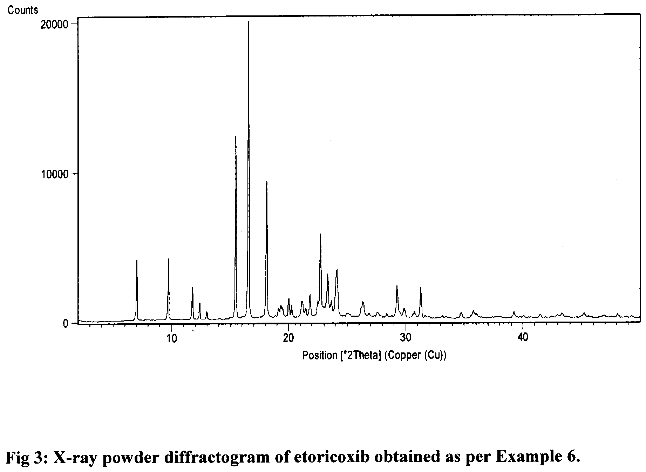

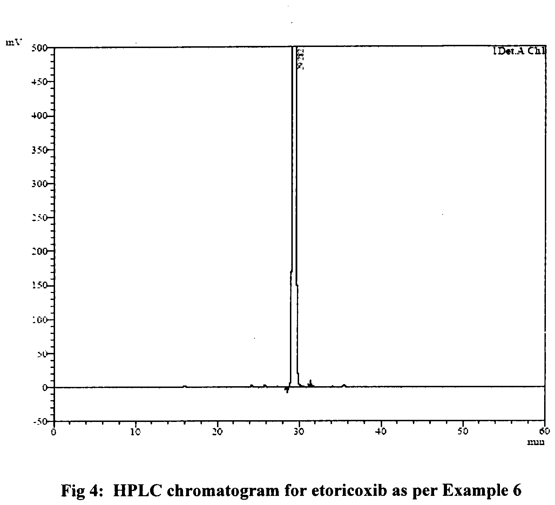

In a 250ml 4-neck round bottom flask equipped with overhead stirrer, double surface condenser, thermowell pocket placed in water bath, 135ml (3volume) of isopropyl alcohol was added followed by 45.0gm etoricoxib crude prepared as per example 5 and heated the reaction mass to about 65°C to about 70°C.The reaction mass was charcoalised with NORIT® charcoal and hot filtered on a hyflo bed and washed with 45 ml (1 volume) hot isopropyl alcohol. The filtered mass was gradually cooled to about 25°Cto about 30°C with continuous stirring and then further cooled to about 0°C to about 5°C and maintained for about an hour and then filtered and washed with 45ml(1 volume) isopropyl alcohol. The wet solids were dried in air oven. Etoricoxib 38gm was obtained with HPLC purity of 99.59% and furan impurity less than 0.1% and particle size distribution: d10=1.32 µm., d50= 4.117 µm., d90 =10.188 µm. Figure 3 shows the XRD pattern of the polymorph of etoricoxib obtained. Figure 4 substantially represents the HPLC chromatograph of etoricoxib.

### Example-7

### Preparation of 5-chloro-3-[4-methylsulfonyl)phenyl]-2-(2-methyl-5-pyridinyl) pyridine, a compound of formula A, etoricoxib.

In a 1L 4-neck round bottom flask equipped with overhead stirrer, double surface condenser, thermowell pocket placed in water bath, 720ml of isopropyl alcohol was added followed by 90.0gm etoricoxib crude prepared as per example 5 and heated the reaction mass to about 65°C to about 70°C.The reaction mass was charcoalised with NORIT® charcoal and maintained at that temperature for about an hour. The reaction mass was hot filtered on a hyflo bed and washed with hot isopropyl alcohol. The filtrate was heated to reflux to obtain a clear solution. The filtrate was gradually cooled to about 25°C to about 30°C with intermittent stirring, wherein the stirring was performed once in every 30 minutes and then further cooled to about 0°C to about 5°C and maintained for about an hour and then filtered and washed with isopropyl alcohol. The wet solids were dried in an air oven. Etoricoxib 80gm was obtained.
HPLC purity: 99.89%
furan impurity less than 0.1% and
particle size distribution: d10=2.64µm., d50= 11.46µm., d90 =54.65 µm.

### Example-8

### Preparation of 5-chloro-3-[4-methylsulfonyl)phenyl]-2-(2-methyl-5-pyridinyl) pyridine, a compound of formula A, etoricoxib.

In a 250ml 4-neck round bottom flask equipped with overhead stirrer , double surface condenser, thermowell pocket placed in water bath, added 100ml (4.0volume) isopropyl acetate followed by 25.0gm etoricoxib crude prepared as per example 5 and heated the reaction mass to about 60°Ca clear solution was obtained.

The reaction mass was charcoalised with 2.5gm NORIT® charcoal and hot filtered on a hyflo bed and washed with 25 ml (1.0 volume) hot isopropyl acetate. The filtrate was again heated to about 60°C to get a clear solution.

To the reaction mass, 125ml (5.0 volume) hexane was slowly added at about 60°C through a dropping funnel. The reaction mass was stirred for 30 min at about 60°C and again 125ml (5.0volume) hexane was added at about the same temperature under stirring. The reaction mass was gradually cooled to about 5°C and the temperature was maintained for 30 min at about 0°C to about 5°C.

The reaction mass was then filtered and washed with 25ml (1:1 isopropyl acetate: hexane). The wet solids 20.6gm were dried in vacuum oven at about 25°C and pure etoricoxib 20gm dried product was obtained.

## Claims

1. A process for the preparation of a compound of formula A, 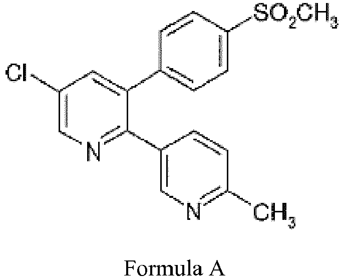 the process comprising reacting a compound of formula II, wherein R1 and R2 are both independently methyl, R3 is chloro and X⁻ is selected from halide or hexafluorphospate, with a compound of formula III A, wherein R4 is methylsulfonyl and R5 is methyl,

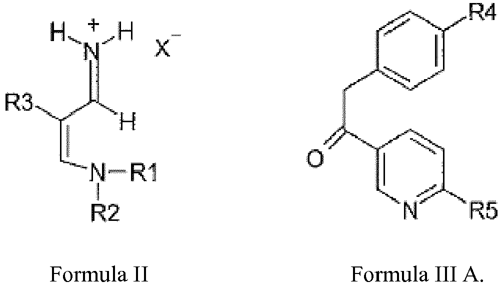

2. The process as claimed in claim 1, wherein the reaction is carried under acidic conditions.

3. A process as claimed in claim 1, wherein the compound of formula A is further purified by a process comprising:
(a) dissolving compound of formula A in tetrahydrofuran to form a solution;
(b) adding water to the above solution; and
(c) isolating the compound of formula A from the solution.

4. A process as claimed in claim 1, wherein the compound of formula A is recrystallized by a process comprising:
(a) adding isopropyl alcohol to compound of formula A to obtain a mixture;
(b) heating the mixture; and
(c) cooling the mixture to obtain compound of formula A.

5. A process as claimed in claim 4, wherein the obtained compound of formula A has less than 0.1% of compound of formula IV, as measured by high performance liquid chromatography

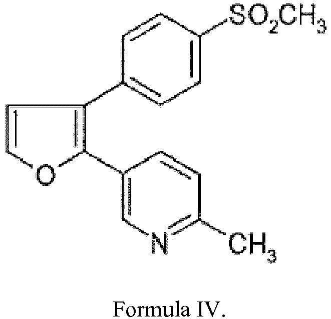

6. A process as claimed in claim 1, wherein the compound of formula A is treated with sodium dithionate.

7. A compound of formula II 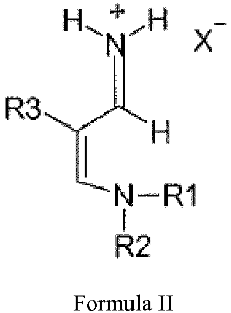 wherein R1 and R2 are independently selected from the group consisting of hydrogen, C₁-C₆ alkyl, aryl, -arylalkyl and -alkylaryl; R3 is halo and X⁻ is a negatively charged counterion.

8. The compound of claim 7, wherein the negatively charged counterion X⁻ is selected from the group consisting of halides, sulfonates, HSO₄⁻ , SO₄²⁻, HCO₃⁻, CO₃²⁻, borate, trifluoroacetate, perchlorate, phosphate, acetate, lactate, succinate, propionate, oxalate, butyrate, ascorbate, citrate, dihydrogen citrate, tartrate, taurocholate, glycocholate, cholate, hydrogen citrate, maleate, benzoate and folate.

9. The compound of claim 8, wherein R1 and R2 are methyl, R3 is chlorine and X⁻ is chloride or hexafluorophosphate.

10. The compound of claim 7 which is 2 chloro-3-(dimethylamino)prop-2-en-1-iminium chloride.

11. Use of 2 chloro-3-(dimethylamino)prop-2-en-1-iminium chloride in the preparation of the compound of formula A.

12. A process for the preparation of the compound of formula II, wherein R1 and R2 are both independently methyl, R3 is chloro and X⁻ is selected from a halide or hexafluorophosphate, the process comprising reacting a compound of formula V, wherein R1 and R2 are as defined above, 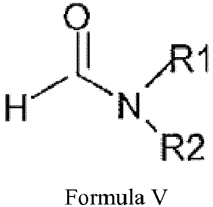 with oxalyl chloride, haloacetyl chloride and ammonia.

## Patentansprüche

1. Verfahren zur Herstellung einer Verbindung nach Formel A, 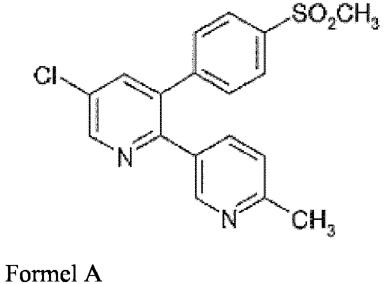 wobei das Verfahren das Reagieren einer Verbindung der Formel II umfasst, wobei R1 und R2 beide unabhängig Methyl sind, R3 Chlor ist und X⁻ ausgewählt ist aus Halogenid oder Hexafluorphosphat, mit einer Verbindung der Formel III A, wobei R4 Methylsulfonyl ist und R5 Methyl ist,

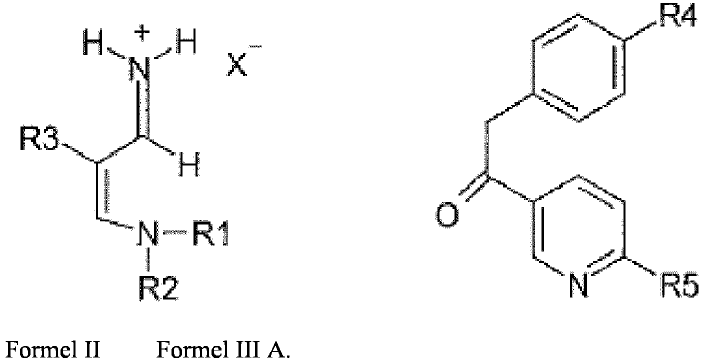

2. Verfahren nach Anspruch 1, wobei die Reaktion unter sauren Bedingungen durchgeführt wird.

3. Verfahren nach Anspruch 1, wobei die Verbindung der Formel A durch ein Verfahren, das Folgendes umfasst, weiter gereinigt wird:
(a) Lösen der Verbindung der Formel A in Tetrahydrofuran zum Bilden einer Lösung;
(b) Zugeben von Wasser zur oben genannten Lösung; und
(c) Isolieren der Verbindung der Formel A aus der Lösung.

4. Verfahren nach Anspruch 1, wobei die Verbindung der Formel A durch ein Verfahren, das Folgendes umfasst, rekristallisiert wird:
(a) Zugeben von Isopropylalkohol zur Verbindung der Formel A, um eine Mischung zu erhalten;
(b) Erwärmen der Mischung; und
(c) Kühlen der Mischung, um die Verbindung der Formel A zu erhalten.

5. Verfahren nach Anspruch 4, wobei die erhaltene Verbindung der Formel A weniger als 0,1 % der Verbindung der Formel IV aufweist, wie durch Hochleistungsflüssigkeitschromatographie gemessen

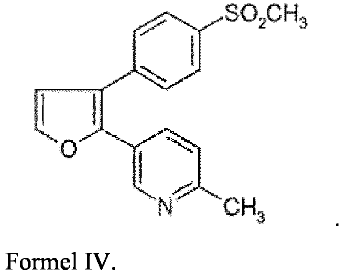

6. Verfahren nach Anspruch 1, wobei die Verbindung der Formel A mit Natriumdithionat behandelt wird.

7. Verbindung der Formel II, 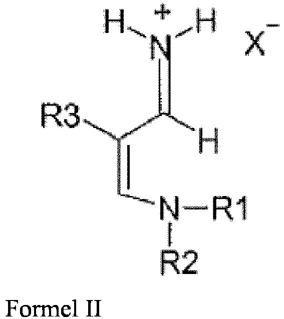 wobei R1 und R2 unabhängig aus der Gruppe ausgewählt sind, die aus Wasserstoff, C₁-C₆-Alkyl, Aryl, -Arylalkyl und -Alkylaryl besteht; wobei R3 Halo ist und X⁻ ein negativ geladenes Gegenion ist.

8. Verbindung nach Anspruch 7, wobei das negativ geladene Gegenion X⁻ aus der Gruppe ausgewählt ist, die aus Halogeniden, Sulfonaten, HSO₄⁻ , SO₄²⁻, HCO₃⁻, CO₃²⁻, Borat, Trifluoracetat, Perchlorat, Phosphat, Acetat, Lactat, Succinat, Propionat, Oxalat, Butyrat, Ascorbat, Citrat, Dihydrogencitrat, Tartrat, Taurocholat, Glycocholat, Cholat, Hydrogencitrat, Maleat, Benzoat und Folat besteht.

9. Verbindung nach Anspruch 8, wobei R1 und R2 Methyl sind, R3 Chlor ist und X⁻ Chlorid oder Hexafluorphosphat ist.

10. Verbindung nach Anspruch 7, die 2-Chlor-3-(dimethylamino)prop-2-en-1 -iminiumchlorid ist.

11. Verwendung von 2-Chlor-3-(dimethylamino)prop-2-en-1-iminiumchlorid bei der Herstellung der Verbindung der Formel A.

12. Verfahren zur Herstellung der Verbindung der Formel II, wobei R1 und R2 beide unabhängig Methyl sind, R3 Chlor ist und X⁻ ausgewählt ist aus einem Halogenid oder Hexafluorphosphat, wobei das Verfahren das Reagieren einer Verbindung der Formel V, wobei R1 und R2 wie oben definiert sind, 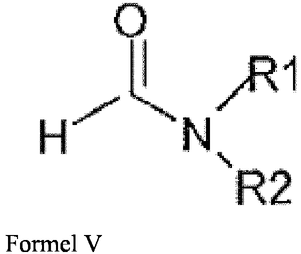 mit Oxalylchlorid, Halogenacetylchlorid und Ammoniak umfasst.

## Revendications

1. Procédé de préparation d'un composé de formule A, 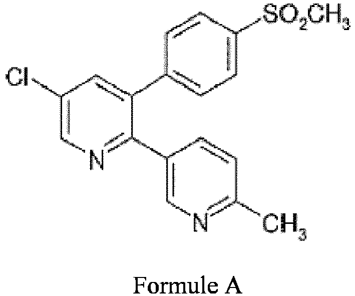 le procédé comprenant la mise en réaction d'un composé de formule II, dans laquelle R1 et R2 représentent tous les deux indépendamment un groupe méthyle, R3 représente un groupe chloro et X⁻ est choisi parmi un halogénure ou un hexafluorophosphate, avec un composé de formule III A, dans laquelle R4 représente un groupe méthylsulfonyle et R5 représente un groupe méthyle,

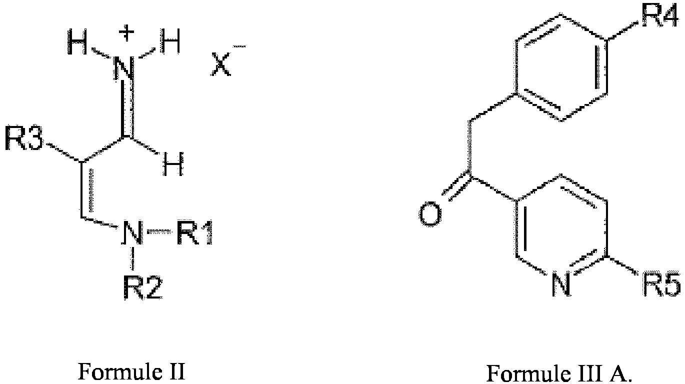

2. Procédé selon la revendication 1, ladite réaction étant effectuée dans des conditions acides.

3. Procédé selon la revendication 1, ledit composé de formule A étant en outre purifié par un procédé comprenant :
(a) la dissolution d'un composé de formule A dans du tétrahydrofurane pour former une solution ;
(b) l'ajout d'eau à la solution précédente ; et
(c) l'isolement du composé de formule A de la solution.

4. Procédé selon la revendication 1, ledit composé de formule A étant recristallisé par un procédé comprenant :
(a) l'ajout d'alcool isopropylique au composé de formule A pour obtenir un mélange ;
(b) le chauffage du mélange ; et
(c) le refroidissement du mélange pour obtenir le composé de formule A.

5. Procédé selon la revendication 4, ledit composé de formule A obtenu comportant moins de 0,1 % du composé de formule IV, tel que mesuré par chromatographie liquide haute performance

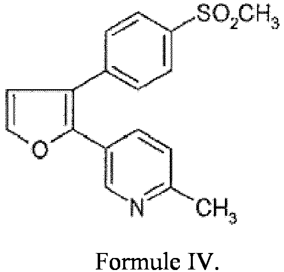

6. Procédé selon la revendication 1, ledit composé de formule A étant traité au dithionate de sodium.

7. Composé de formule II 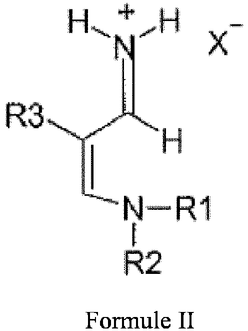 dans laquelle R1 et R2 sont indépendamment choisis dans le groupe constitué par un atome d'hydrogène, un groupe (C₁ à C₆)alkyle, un groupe aryle, un groupe -arylalkyle et un groupe -alkylaryle ; R3 représente un groupe halogéno et X⁻ représente un contre-ion chargé négativement.

8. Composé selon la revendication 7, ledit contre-ion chargé négativement X⁻ étant choisi dans le groupe constitué par les halogénures, les sulfonates, HSO₄⁻, SO₄²⁻, HCO₃⁻, CO₃²⁻, le borate, le trifluoroacétate, le perchlorate, le phosphate, l'acétate, le lactate, le succinate, le propionate, l'oxalate, le butyrate, l'ascorbate, le citrate, le dihydrogénocitrate, le tartrate, le taurocholate, le glycocholate, le cholate, l'hydrogénocitrate, le maléate, le benzoate et le folate.

9. Composé selon la revendication 8, R1 et R2 représentant un groupe méthyle, R3 représentant un atome de chlore et X⁻ représentant un ion chlorure ou un hexafluorophosphate.

10. Composé selon la revendication 7 qui est le chlorure de 2 chloro-3-(diméthylamino)prop-2-én-1-iminium.

11. Utilisation de chlorure de 2 chloro-3-(diméthylamino)prop-2-én-1-iminium dans la préparation du composé de formule A.

12. Procédé de préparation du composé de formule II, dans laquelle R1 et R2 représentent tous les deux indépendamment un groupe méthyle, R3 représente un groupe chloro et X⁻ est choisi parmi un halogénure ou un hexafluorophosphate, le procédé comprenant la mise en réaction d'un composé de formule V, dans laquelle R1 et R2 sont tels que définis précédemment, 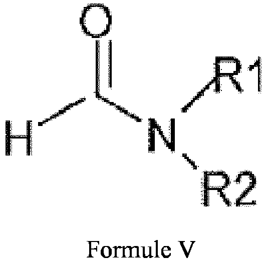 avec du chlorure d'oxalyle, du chlorure d'halogénoacétyle et de l'ammoniaque.
